(19) Europäisches Patentamt / European Patent Office / Office européen des brevets

(11) **EP 2 075 019 B1**

(12) **EUROPEAN PATENT SPECIFICATION**

(45) Date of publication and mention
of the grant of the patent:
**07.03.2012 Bulletin 2012/10**

(21) Application number: **07829816.3**

(22) Date of filing: **15.10.2007**

(51) Int Cl.:
**A61M 1/34** [(2006.01)]

(86) International application number:
**PCT/JP2007/070082**

(87) International publication number:
**WO 2008/047760 (24.04.2008 Gazette 2008/17)**

(54) **BIOARTIFICIAL RENAL TUBULE**

BIOARTIFIZIELLER NIERENTUBULUS

TUBULE RÉNAL BIOARTIFICIEL

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **17.10.2006 JP 2006282826**

(43) Date of publication of application:
**01.07.2009 Bulletin 2009/27**

(73) Proprietor: **TOKAI UNIVERSITY EDUCATIONAL SYSTEM**
**Tokyo 1510063 (JP)**

(72) Inventors:
• **SAITO, Akira**
  **Isehara-shi**
  **Kanagawa 259-1193 (JP)**
• **YOKOYAMA, Tun Aung**
  **Isehara-shi**
  **Kanagawa 259-1193 (JP)**

(74) Representative: **Michalski Hüttermann & Partner**
**Patentanwälte**
**Neuer Zollhof 2**
**40221 Düsseldorf (DE)**

(56) References cited:
WO-A1-89/01967          WO-A1-2005/063147
JP-A- 2000 271 213     JP-A- 2004 267 795
US-A1- 2005 238 687

• INAGAKI ET AL: "Prevention of LLC-PK1 cell overgrowth in a bioartificial renal tubule device using a MEK inhibitor, U0126", JOURNAL OF BIOTECHNOLOGY, ELSEVIER SCIENCE PUBLISHERS, AMSTERDAM, NL, vol. 132, no. 1, 5 October 2007 (2007-10-05), pages 57-64, XP022285748, ISSN: 0168-1656, DOI: DOI: 10.1016/J.JBIOTEC.2007.08.025
• LI S ET AL: "Evidence for ERK1/2 phosphorylation controlling contact inhibition of proliferation in Madin-Darby canine kidney epithelial cells", AMERICAN JOURNAL OF PHYSIOLOGY. CELL PHYSIOLOGY, AMERICAN PHYSIOLOGICAL SOCIETY, US, vol. 287, no. 2, 1 August 2004 (2004-08-01), pages C432-C439, XP003022207, ISSN: 0363-6143, DOI: DOI: 10.1152/AJPCELL.00020.2004
• LI ET AL.: 'Evidence for ERK1/2 phosphorylation controlling contact inhibition of proliferation in Madin-Darby canine kidney epithelial cells' AM. J. PHYSIOL. CELL PHYSIOL. vol. 287, no. 2, August 2004, pages C432 - C439, XP003022207
• FUJITA Y. ET AL.: 'Evaluation of Na+ active transport and morphological changes for bioartificial renal tubule cell device using Madin-Darby canine kidney cells' TISSUE ENGINEERING vol. 8, no. 1, February 2002, pages 13 - 24, XP003022208

EP 2 075 019 B1

**Description**

Technical Field

The present invention relates to a bioartificial renal tubule, that is, an artificial renal tubule containing a confluent monolayer of renal tubular epithelial cells and also relates to a method of maintaining of a monolayer of the cells.

Background Art

Aebischer et al. were the first to develop a bioartificial renal tubule using an artificial membrane and renal tubular epithelial cells and released a basic study on the bioartificial renal tubule in 1987 (Non-patent Documents 1 to 3). However, the study was suspended in 1989. In 1998, Humes et al. at University of Michigan reported that a bioartificial renal tubule prepared by grafting proximal renal tubular epithelial cells onto the inner surface of a polysulfone hollow fiber was capable of inhibiting the leakage of inulin and had various metabolic functions due to almost complete adhesion of the cells (Non-patent Document 4). Humes et al. succeeded in extracorporeal circulation experiments on renal failure dogs using bioartificial renal tubule devices (Non-patent Document 5) and performed the first clinical application of a bioartificial renal tubule device to an acute renal failure patient with endotoxin-induced sepsis in 2001 (Non-patent Document 6). Humes et al. treated ten acute renal failure patients with multiple organ failure in 2004 and then reported that six of the patients were improved in life prognosis (Non-patent Document 7). In the above experiments and clinical applications, the bioartificial renal tubule devices were used only for 24 hours and were not used for more than 24 hours for long-term treatments.

The inventors have cultured various renal tubular epithelial cells on flat membranes and in hollow fiber membrane modules over a long term to develop a bioartificial renal tubule device which protects a chronic dialysis patient from complications and which is capable of maintaining its performance for a long period of time. From long-term culture, the inventors have confirmed that renal tubular epithelial cells isolated from kidney tissues cannot maintain contact inhibition which enables the cells to form a monolayer cylindrical renal tubule in a kidney tissue because the renal tubular epithelial cells lack interactions with other cells in the kidney tissue or are varied in properties during culture. The inventors have found that in long-term observation, renal tubular epithelial cell lines, which have been supposed to stop multiplying because of contact inhibition after these cells form confluent monolayers, are stratified after these cells form the confluent monolayers (Non-patent Documents 8 and 9). The inventors have also found that a renal tubule device seriously deteriorates in performance because cells used are stratified one to two weeks later after the cells form a confluent monolayer depending on the type of the cells. In the case where a large number of artificial renal tubule devices are prepared and supplied to renal failure patients, each artificial renal tubule device needs to wait for a while until the artificial renal tubule device is required for treatment. For this while, the stratification of cells used causes the deterioration of the artificial renal tubule device. When the artificial renal tubule devices need to be used to repeatedly treat a chronic patient, the cells are stratified during cultivation on treatment intervals and therefore the artificial renal tubule devices are deteriorated in performance.

[List of references]

Non-patent Document 1: Aebischer P, Ip TK, Miracoli L, Galletti PM, Renal epithelial cells grown on semipermeable processor, Trans. Am. Soc. Artif. Intern. Organs, 33, 96-102, 1987
Non-patent Document 2: Aebischer P, Ip TK, Miracoli L, Galletti PM, The bioartificial kidney: Progress toward an ultrafiltration device with renal epithelial cells processing, Life Support Sys., 5, 159-168, 1987
Non-patent Document 3: Ip TK, Aebischer P, Renal epithelial cell-controlled solute transport across permeable membranes as the foundation for a bioartificial kidney, Artif. Organs, 13, 58-61, 1987
Non-patent Document 4: Humes HD, MacKay SM, Funke AJ, Buffington DA, Tissue engineering of a bioartificial renal tubule assist device: in vitro transport and metabolic characteristics, Kidney Int., 55, 2502-2514, 1999
Non-patent Document 5: Humes HD, Buffington DA, MacKay SM, Funke AJ, Weitzel WF, Replacement of renal function in uremic animals with a tissue-engineered kidney, Nat. Biotechnol., 17, 451-455, 1999
Non-patent Document 6: Weitzel WF, Fissel WH, Humes HD, Initial clinical experience with a human proximal tubule cell renal assist device (RAD), J. Am. Soc. Nephrol, 12 (Program and Abstracts issue), 279A, 2001
Non-patent Document 7: Humes HD, Weitzel WF, Barlett RH, Swaniker FC, Paganini EP, Luderer JR, Sobota J, Initial clinical results of the bioartificial kidney containing human cells in ICU patients with acute renal failure, Kidney Int. 2004, 66, 1587-1588
Non-patent Document 8: Fujita Y, Kakuta T, Saito A, et al., Evaluation of Na+ active transport and morphological changes for bioartificial renal tubule cell device using Madin-Dar by canine kidney cells, Tissue Eng. 8, 13-24, 2002
Non-patent Document 9: Ozgen N, Tarashima M, Aung T, Sato Y, Isoe C, Kakuta T, Saito A, Evaluation of long-term transport ability of a bioartificial renal tubule device using LLC-PK1, Nephrol Dial Transplant 2004, 19, 2198-2207

A bioartificial renal tubule of the prior art is disclosed in JP-2004-267795, corresponding to WO-95/11048.

Disclosure of Invention

[Problems to be Solved by the Invention]

In order to develop a bioartificial renal tubule which is capable of faithfully reproducing the reabsorption function of a kidney and which includes an artificial membrane and renal tubular epithelial cells grafted on the inner surface of the artificial membrane, the inventors have investigated problems such as functional reduction and deterioration in view of the status of the above bioartificial renal tubules and have made intensive studies. As a result, the inventors have succeeded in continuously forming a confluent monolayer of renal tubular epithelial cells on the inner surface of an artificial membrane in such a manner that a MEK (mitogen activated protein kinase) inhibitor is used to maintain the contact inhibition of the renal tubular epithelial cells. The inventors have found that cellular functions can be maintained over a long period in such a manner. This has led to the completion of the present invention, which provides a device that forms an artificial kidney having a function more similar to that of a vital kidney together with a bioartificial glomerulus functioning as a hemofilter.

[Means for Solving the Problems]

A bioartificial renal tubule according to the present invention includes an artificial membrane having renal tubular epithelial cells attached to the inner an outer surface thereof and a vessel containing the artificial membrane. This bioartificial renal tubule is characterized by that the cells are prevented by the use of a MEK inhibitor from being stratified and therefore form a confluent monolayer on the artificial membrane.
In the bioartificial renal tubule, the renal tubular epithelial cells may exhibit contact inhibition because of the use of the MEK inhibitor.
The renal tubular epithelial cells may be RPTEC or other primary cultured renal tubular epithelial cells, MDCK cells, LLC-PK$_1$ cells, JTC-12 cells, or HK-2 cells.
The MEK inhibitor may be at least one selected from the group consisting of U0126, PD98059, and CI-1040. The MEK inhibitor is preferably U0126.
The artificial membrane may be formed from a hollow fiber membrane made of polysulfone, cellulose acetate, or polyimide.
A method of maintaining a confluent monolayer of renal tubular epithelial cells in a bioartificial renal tubule according to the present invention is characterized, according to claim 7, in including applying a MEK inhibitor to renal tubular epithelial cells such that the cells exhibit contact inhibition and preventing the stratification of the cells after the cells form the confluent monolayer on the inner surface of the artificial membrane.
The MEK inhibitor may be used in such a manner that the MEK inhibitor is added to a maintenance broth for culturing the renal tubular epithelial cells such that the concentration of the MEK inhibitor in the maintenance broth is 30 to 50 $\mu$M.

Advantages

[Effect of the invention]

The bioartificial renal tubule according to the present invention aims to faithfully reproduce the reabsorption function of a kidney and is an artificial renal tubule device which is prepared by attaching a confluent monolayer of renal tubular epithelial cells to the inner surface of an artificial membrane so as to efficiently and selectively reabsorb a useful substance, an electrolyte, and water.
The following problem can be prevented over a long period in such a manner that a MEK inhibitor is used to maintain the contact inhibition of the renal tubular epithelial cells: a problem that other renal tubular epithelial cells overlap a renal tubular epithelial cells and therefore active transport is prevented or a problem that the renal tubular epithelial cells are stratified and therefore lumens of the artificial membrane are narrowed so that the flow of primitive urine is interrupted and therefore the reabsorption of useful substances. This allows the function of the renal tubular epithelial cells, which are attached to the inner surface of the artificial membrane in the form of a confluent monolayer, to be maintained.

The bioartificial renal tubule according to the present invention is a device which forms an artificial kidney together with a bioartificial glomerulus. The bioartificial renal tubule is useful in providing a safe, simple sustainable treatment system that prevents a disadvantage due to the continuous use of an anticoagulant agent such as heparin when blood needs to be continuously filtered to improve symptoms, such as chronic or acute cardiac failure, chronic or acute renal failure, and multiple organ failure, causing overhydration or accumulation of metabolites.

Detailed Description of the Invention

Blood is filtered through glomeruli of kidneys, so that substances other than blood cells and protein move into renal tubules in the form of solutes in "primitive urine", which is a filtrate. Necessary substances contained in primitive urine are reabsorbed into the blood through the renal tubules but waste products are discarded. Then urine is formed and discharged from a body. Vital kidneys have a metabolic function, a function of adjusting blood pressure and electrolyte concentration, a function of adjusting acid-base equilibrium, and an endocrine function and various types of complicated cells are involved in these functions; hence, kidney is probably one of the least regenerative organs.

Artificial kidneys currently used and treatments using the artificial kidneys have been technically established; however, the artificial kidneys are far inferior in function to vital kidneys and merely partially carry out the removal of body wastes and electrolyte adjustment only. That is, the artificial kidneys have low substance permeation selectivity and therefore eliminate useful substances such as glucose, amino acids, hormones, and ions. The metabolic function and the endocrine function of the vital kidneys are not substituted by that of artificial kidneys currently used, hence patients suffer from various complications. Therefore, it is essential to develop a continuous, efficient system suitable for next-generation artificial kidney treatments because of the above reasons and the inconvenience of intermittent hemodialysis. Novel systems for artificial kidney treatments such as ambulatory artificial kidney treatments and dialysate generation-type peritoneal dialysis are under development. Furthermore, a bioartificial kidney system including glomerular/renal tubular cells derived from a patient or cells having similar functions enhanced by the introduction of genes is ultimately targeted.

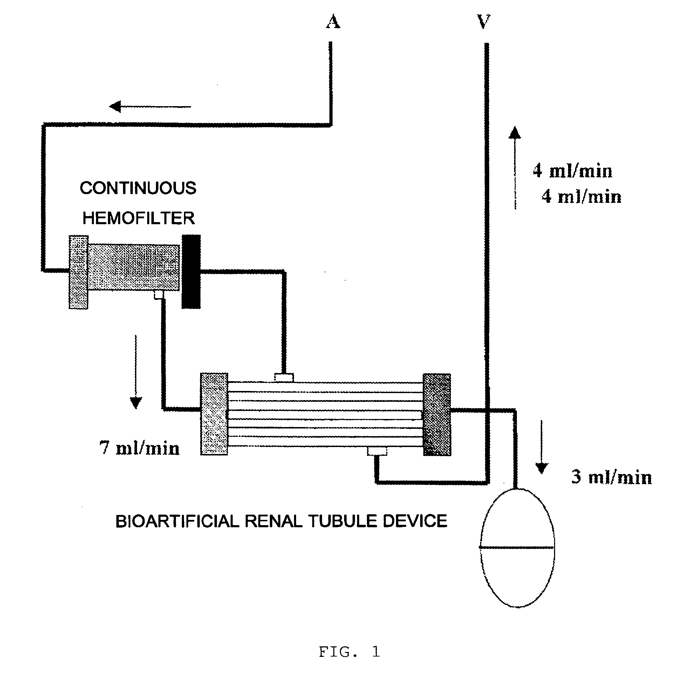

The term "artificial renal tubule" means a renal tubule device artificially carrying out renal tubular reabsorption and metabolism. This renal tuble device has a function similar to the reabsorption function of renal tubules recovering glucose, amino acids, ions, and water from primitive urine, acting as a kidney. The term "bioartificial renal tubule" means a combination of such an artificial renal tubule with cells, tissues, and a biomaterial. The term "bioartificial kidney" means a system including a bioartificial glomerulus, bioartificial renal tubules, and an antithrombogenic circuit (which may include a liquid transfer pump) connecting the bioartificial glomerulus and the bioartificial renal tubules. This system can continuously administer a kidney-alternative treatment for 24 hours with high efficiency owing to using cells that can reproduce the function of a kidney. The useful substance-reabsorbing function of renal tubules can be added to this system. Fig. 1 is a schematic view of a target bioartificial kidney proposed by the inventors. Artificial kidneys currently used for dialysis treatments perform rapid dialysis within a limited time and have limited effects.

- Bioartificial renal tubule

The bioartificial renal tubule according to the present invention inclueds an artificial membrane having renal tubular epithelial cells attached to the inner or outer surface thereof and a vessel containing the artificial membrane. This bioartificial renal tubule is characterized by that the cells are prevented by the use of a MEK inhibitor from being stratified and therefore form a confluent monolayer on the artificial membrane.

The renal tubular epithelial cells are attached to the inner surface of a hollow fiber placed in the artificial membrane. This allows the bioartificial renal tubule to have a selective substance-reabsorbing function similar to that owned by a vital kidney. The following selectivity can be achieved by the use of the renal tubular epithelial cells: the selectivity that glucose, ions, water, and the like are absorbed from a plasma filtrate (or primitive urine flowing through a nephron renal tubule) flowing in an inner portion of the artificial membrane (or a lumen of the hollow fiber) but ammonia, creatinine, drugs, waste products, and the like are not absorbed. That is, low-molecular-weight useful substances are filtered into a dialysate from a blood stream by the sieving action of a glomerulus and are then reabsorbed into the blood stream by the active transport of a substance by a renal tubule.

For the preparation of a bioartificial organ, the following techniques are important: a technique for improving the affinity of an artificial material, used as a scaffold, to cells and a technique for allowing cells which are isolated from a vital organ or tissue and which are placed on a scaffold of an artificial material to exhibit the same function as that of the vital organ. In the bioartificial renal tubule, the formation and maintenance of a monolayer, as well as the function of selected and/or taken cells, is important to allow the renal tubular epithelial cells, which have polarity on mass transfer, to function as a renal tubule. The renal tubular epithelial cells exhibit "contact inhibition" (a feature that the growth of a cell is arrested when the cell comes into contact with other grown cells all around), which is a feature of normal cells to form and maintain a monolayer when being present in a kidney tissue.

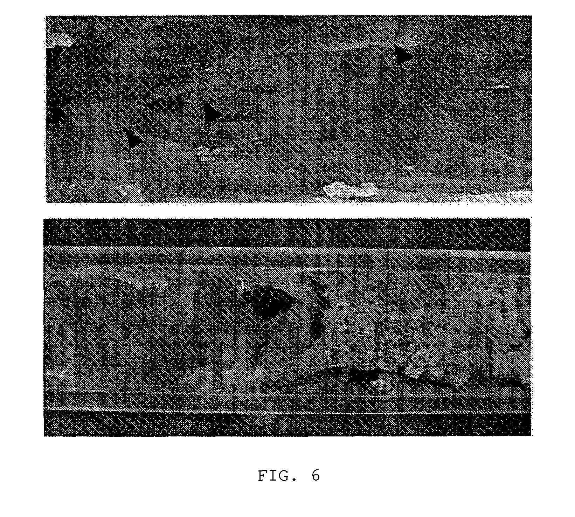

Studies performed by the inventors have shown that the renal tubular epithelial cells isolated from a tissue form a confluent monolayer and are then stratified and this gradually reduces the metabolic function and transport function of a renal tubule. The inventors have reported that MDCK cells and LLC-PK$_1$ cells, which are cell lines, chronically cultured lack contact inhibition; hence, cells overlap cells two weeks later after the formation of confluent monolayers to cause stratification (Non-patent Documents 8 and 9). Furthermore, the inventors have reported that the above stratification reduces the ability to transport water, glucose, sodium, and the like. The inventors have confirmed that RPTECs, which are primary cultured proximal renal tubular epithelial cells, lack contact inhibition during long-term culture and therefore are stratified (Fig. 6). The overlap of grown renal tubular epithelial cells on a monolayer, that is, the stratification thereof interferes with the flow of primitive urine and reduces the efficiency of reabsorption of useful substances. The term "confluent", which is involved in cell density, means a state in which neighboring cells are in contact with each other and are densely packed.

- Artificial membrane

The bioartificial renal tubule according to the present invention includes the artificial membrane, which has the renal tubular epithelial cells attached thereto, and the vessel, which contains the artificial membrane. The artificial membrane needs to be porous. Filtration membranes such as hollow fiber membranes are used for hemodialysis because of its various advantages, an artificial hollow fiber membrane is also used as the artificial membrane included in the bioartificial renal tubule according to the present invention.

- Hollow fiber

The bioartificial renal tubule according to the present invention preferably includes a hollow fiber membrane which has micropores uniformly distributed and also has an extremely large support capacity. The hollow fiber membrane is widely used for filtration and material separation. A large number of the micropores are present in a side surface of the hollow fiber membrane that is located between the inner and outer surfaces of the hollow fiber membrane. The micropores preferably have a size of 0.001 to several micrometers and more preferably 0.03 to 1 $\mu$m. The membrane includes a hollow fiber and therefore is extremely greater in support capacity or accommodation capacity per unit surface area as compared to other porous materials. Therefore, the membrane can be used as a support, having an extremely great ability, suitable for use in a fine medical device.

Examples of a material used to form the hollow fiber include polysulfone, polyethersulfone, polyacrylonitrile, polyvinyl alcohol, and cellulose acetate. A preferable base material may be any one of hollow fiber membranes for hemodialysis, and synthesized macromolecule hollow fiber membranes for artificial kidneys are preferably used. The hollow fiber is desirably formed from a film made of cellulose acetate, polysulfone, polyimide, or an ethylene vinylalcohol copolymer.

Japanese Patent No. 2916446 discloses a method of producing an asymmetric microporous hollow filament suitably used as an ultrafiltration hollow fiber for hemodialysis. The hollow filament has such a continuous porous sponge structure that micropores increase in size outward and also has an inner surface having a microporous barrier layer thereon. Therefore, the hollow filament is available for artificial kidneys, filters for artificial hemodialysis, and the like. The hollow filament has excellent fluid permeability, mechanical strength, and workability and therefore is a preferred material for forming the hollow fiber membrane of the present invention.

- Renal tubular epithelial cells

The renal tubular epithelial cells, which are attached to the inner surface of the hollow fiber in the form of a confluent monolayer, are not particularly limited and various types of renal tubular epithelial cells may be used herein. For example, epithelial cells of a human renal tubule taken from a kidney anatomically unsuitable for transplantation may be used. The renal tubular epithelial cells may be derived from cultured epithelial cell lines, gene-introduced substitute epithelial cells, pluripotent stem cells, or similar cells. More specifically, examples of the renal tubular epithelial cells include human renal tubular epithelial cells, Madin-Darby canine kidney (MDCK) cells, human proximal tubular cell lines HK-2, JTC-12 cells, LLC-PK$_1$ cells, and primary cultured human proximal renal tubular epithelial cells (RPTECs). In practice, a suitable renal tubular epithelial cells is selected desirably in view of cell grafitability, active transport ability, metabolizability, duration of forming monolayer, and the like.

The use of cell engineering technology and gene engineering technology making great progress allows the separation, culture, and multiplication of differentiated renal tubular epithelial precursor cells derived from stem cells and the induction of the differentiated renal tubular epithelial precursor cells into more differentiated renal tubular epithelial cells. Cells enhanced in similar function by gene introduction may be used.

To attach the renal tubular epithelial cell to the inner surface of the artificial membrane in the form of a confluent monolayer means to graft the cells, preferably to adhere the cells in a form of a confluent monolayer, onto the inner surface of the artificial membrane. The inner surface of the artificial membrane may be coated with the cells. A technique for attaching the renal tubular epithelial cells to the inner surface of the artificial membrane is not particularly limited. The renal tubular epithelial cells may be adsorbed on, bonded to, or supported on the inner surface thereof so as not to be liberated into a liquid but so as to be fixed. The inner surface of the artificial membrane is where a plasma filtrate flows, which corresponds to a lumen of a hollow fiber placed in a hollow fiber membrane which is a preferred example of the artificial membrane. The renal tubular epithelial cells may be attached to the outer surface of the artificial membrane as required. If the renal tubular epithelial cells are attached to the inner surface (lumen) of the hollow fiber membrane, the renal tubular epithelial cells are attached to the inner wall of the lumen of the hollow fiber. The inner wall of the lumen has a large number of micropores communicatively connected to the outside of the hollow fiber. Water, electrolytes, and useful substances selectively pass through the micropores to move into blood flowing outside the hollow fiber. Although the micropores are covered with the confluent monolayer of the renal tubular epithelial cells and therefore are blocked, the active transport ability of the renal tubular epithelial cells allows the control of selective movement. A particular technique is as described below. For the use of, for example, a polysulfone hollow fiber membrane device (including 1600 hollow fibers with an inner diameter of 300 nm) with a membrane area of 0.4 m$^2$, LLC-PK$_1$ renal tubular epithelial cells are seeded in the hollow fibers at a density of 10$^6$ cells/mL four times every hour, whereby a confluent monolayer is formed within 24 hours. This has been confirmed by the inventors.

An extracellular matrix may be attached to the hollow fiber in addition to the renal tubular epithelial cells. Examples of the extracellular matrix include Collagen I, Collagen IV, laminin, fibronectin, and Pronectin. The application of these matrices to the hollow fiber will promote the renal tubular epithelial cells to be fixed on the inner surface of the hollow fiber. These cell-adhesive proteins and adhesive polymers are used in such a manner that they are applied to the hollow fiber in advance of seeding cells.

- Drug

In the bioartificial renal tubule according to the present invention, the MEK inhibitor is used to maintain the contact inhibition of the renal tubular epithelial cells. The MEK inhibitor may be at least one selected from the group consisting of U0126, PD98059, and Cl-1040. These drugs may be used alone or in combination. The MEK inhibitor is preferably U0126, which inhibits the activation of a MAP kinase kinase (MEK) and suppresses the activity of ERK 1/ERK 2. The MEK inhibitor is described below in detail.

Mitogen-activated protein kinase (MAPK) pathways are involved in cell functions such as growth, differentiation, and stress response. These pathways are linear kinase cascades. In particular, a MAP kinase kinase kinase phosphorylates and activates a MAP kinase kinase that phosphorylates and activates a MAP kinase. The following congeners and members have been identified at present: seven MAP kinase congeners, that is, MEK1, MEK2, MKK3, MKK4/SEK, MEK5, MKK6, and MKK7 and four MAPK family members, that is, ERK1/2, JNK, p38, and ERK5. The activation of these pathways controls the activity of various substrates through phosphorylation. The substrates include p62$^{TCF}$(Elk-1), c-Myc, and ATF2 and also include c-Fos and c-Jun, which are AP-1 factors.

U0126, which has the chemical name 1,4-diamino-2,3-dicyano-1,4-bis[2-aminophenylthio]butadiene ($C_{18}H_{16}N_6S_2$) , has been developed through studies on the suppression of cell proliferation in the field of cancer and its action, that is, the suppression of the expression of MEK has been studied. U0126, which is an ERK/MEK inhibitor, has been identified as a suppressor of AP-1 transactivation in a cell-based reporter assay. It has been reported that U0126 suppresses an endogenous promoter containing an AP-1 response molecule and has no influence on a gene containing a promoter that lacks an AP-1 response element. These effects of U0126 result from the direct inhibition of the MAPK family members: MEK-1 and MEK-2. However, U0126 shows little effect on the kinase activity of protein kinase C, Abl kinase, Raf, MEKK, ERK, JNK, MKK-3, MKK-4/SEK, MKK-6, Cdk2, or Cdk4 (Favata MF, Horiuchi KY, Manos EJ, Daulerio AJ, Stradley DA, Feeser WS, Van Dyk DE, Pitts WJ, Earl RA, Hobbs F, Copeland RA, Magolda RL, Scherle PA, and Trzaskos JM, Identification of a novel inhibitor of mitogen-activated protein kinase kinase, J. Biol. Chem. 1998, 273, 18623-18632).

Epithelial cell systems are supposed to have such a feature (contact inhibition) that the proliferation thereof is arrested when the epithelial cell systems come into contact with proliferated cells all around. Li et al. have shown that a MEK inhibitor allows Madin-Darby canine kidney (MDCK) cells to exhibit contact inhibition when the cells lack such a feature because of the action of a hepato-growth factor (HGF) (Li S, Gerrard ER Jr., and Balkovetz DF, Evidence for ERK1/2 phosphorylation controlling contact inhibition of proliferation in Madin-Darby canine kidney epithelial cells, Am. J. Physiol Cell Physiol, 2004, 287, C432-C439). It has been shown that U0126 and PD-98059 have a function of restoring the contact inhibition of the MDCK cells; however, this is involved in a mechanism of controlling cell density depending on proliferation. There is no description about the maintenance of cell monolayers.

The inventors have confirmed that MDCK cells, porcine proximal renal tubular epithelial LLC-PK$_1$ cells, human proximal tubular cell lines HK-2, and RPTECs, which are primary cultured proximal renal tubular epithelial cells grow on cell layers two weeks later after the formation of the confluent monolayers to cause stratification.

In consideration of providing an artificial renal tubule device to a patient when the device is medically required, it is too late that the device is manufactured at the point of time when the device is required. Hence, the device needs to be manufactured in advance and also needs to be preserved until being delivered. However, since stratification starts at the point of time when a confluent monolayer is formed, the device can have been apparently deteriorated in function at the point of time when the device is used to treat the patient. Therefore, the device is desirably received by the patient in such a state that the confluent monolayer is maintained until just before the treatment of the patient. For the use of the device for intermittent bioartificial kidney treatment, the MEK inhibitor needs to be added to the device during intervals between treatments such that the confluent monolayer is maintained. It has been shown that after the addition of the MEK inhibitor is stopped, ERK mRNA is immediately expressed and proliferated cells return to a pre-use state. It has been shown that the confluent monolayer can be maintained for a long time by maintaining contact inhibition. Therefore, bioartificial renal tubules and bioartificial glomerular devices which are manufactured in advance and are in a standby condition are allowed to restore their original functions in such a manner that the MEK inhibitor is added to the maintenance broth resulting in an appropriate concentration and the maintenance broth is replaced with a standard broth in advance of use.

Studies performed by the inventors have yielded findings useful in carrying out the present invention as described below.

(1) Renal tubular epithelial cells of mammalians such as canine, porcine, and human overlap the confluent monolayer to cause stratification two weeks later after the formation of the confluent monolayer on an artificial membrane even though the cells are cell lines or primary cultured cells. (2) The contact inhibition of epithelial cells is maintained and a monolayer thereof is maintained in such a manner that the MEK inhibitor is added to a medium such that the concentration of the MEK inhibitor in the medium is 30 to 50 $\mu$M. (3) The non-use of the MEK inhibitor causes the loss of such an action to recover the proliferation activity of cells. Furthermore, the inventors have confirmed the fact that primary cultured human proximal renal tubular epithelial cells form a confluent monolayer and are then stratified during further culture (date not shown)..

- Technique for preventing the stratification of renal tubular epithelial cells

The inventors have investigated conditions that overcome the above stratification and that allow renal tubule devices to be timely delivered in such a state that the devices are protected from stratification. This issue applies to the functional maintenance of continuous hemofilters (which may be referred to as bioartificial glomeruli) prepared by attaching endothelial cells, having no polarity on mass transfer, to surfaces of artificial membranes such as hollow fibers in view of that a formed monolayer needs to be maintained for a long time for the functional maintenance thereof although no substance is actively transported.

In order to obtain cells necessary for bioartificial renal tubule devices, renal tubular epithelial cells are cultured in bulk. An obtained emulsion containing the cultured cells is seeded in a hollow fiber module. After the renal tubular epithelial cells form a confluent monolayer, the active transport of metabolites is reduced because the cells over-confluent (Non-patent Documents 8 and 9). This is because the proliferation and stratification of the cells prevents directional active transport and the swelling of the cells narrows lumens of a hollow fiber. The hollow fiber lumens are blocked with clusters of the cells in some cases.

In order to allow the seeded renal tubular epithelial cells to exhibit contact inhibition to suppress the proliferation of the cells, an inhibitor of an extracellular-signal regulated kinase kinase (MAPK kinase) (MEK), for example, U0126 needs to be added to a broth for culturing the cells. The MEK inhibitor U0126 suppresses the MAPK kinase-induced phosphorylation of threonine and tyrosine to inhibit MAPK (mitogen-activated protein kinase), which is one of ERKs, from acting on the cell cycle of MAP (mitogen-activated protein). However, U0126 has no influence on cellular activities (protein kinase C, Abl kinase, Raf, JNK, MKK, and Cdk) other than the MAPK pathway and therefore is supposed to be safe (Favata MF, Horiuchi KY, Manos EJ, Daulerio AJ, Stradley DA, Feeser WS, Van Dyk DE, Pitts WJ, Earl RA, Hobbs F, Copeland RA, Magolda RL, Scherle PA, and Trzaskos JM, Identification of a novel inhibitor of mitogen-activated protein kinase kinase, J. Biol. Chem. 1998, 273, 18623-18632 and Dudley DT, Pang L, Decker SJ, Bridges AJ, and Saltiel AR, Proc. Natl. Acad. Sci. USA, 1995, 92, 7868-7889). The inventors have evaluated effects on the suppression of cell proliferation and the formation and maintenance of confluent monolayers by using LLC-PK$_1$ cells, which are porcine proximal renal tubular epithelial cells, and adding U0126 to their broths and also have evaluated influences on the proliferation of the cells free from U0126 as described below.

Accordingly, in the bioartificial renal tubule of the present invention, the method for maintaining a confluent monolayer of renal tubular epithelial cells is characterized in that a MEK inhibitor is used to allow the renal tubular epithelial cells to exhibit contact inhibition and the cells are thereby prevented from being stratified after the cells form a confluent monolayer on the inner surface of an artificial membrane. The formed confluent monolayer is preferably maintained continuously for at least two weeks or more, more preferably three weeks, and further more preferably four weeks by the method according to the present invention.

In the method, the MEK inhibitor is used in such a manner that the MEK inhibitor is added to a maintenance broth for maintaining the bioartificial renal tubule and bioartificial glomerular device, which are manufactured in advance and are in a standby condition, such that the concentration of the MEK inhibitor in the maintenance broth is 20 to 100 $\mu$M, preferably 30 to 50 $\mu$M, whereby the cells are prevented from being stratified. The maintenance broth is replaced with a standard broth in advance of use, whereby an original function can be restored.

In the case where the bioartificial renal tubule device is used in combination with current intermittent hemodialysis (filtration), the stratification of the cells can be prevented by using the MEK inhibitor to culture the cells during intervals between treatments.

- Renal tubule vessel

Current hemofiltration devices are used in the form of a type of cartridge including a filtration device containing a hollow fiber filtration membrane. The bioartificial renal tubule according to the present invention is preferably of such a cartridge type. Therefore, a renal tubule vessel for containing the hollow fiber membrane may be made of the same material as that used to form conventional cartridges. When the bioartificial renal tubule is of a wearable or embedded type for the purpose of continuous hemofiltration, it is preferred that the bioartificial renal tubule has a configuration suitable for the purpose, be more compact, and be made of a biocompatible material. The vessel not only contains the artificial membrane but also includes necessary components such as a structural component for fixing and supporting the artificial membrane and structural components such as inlets and outlets for liquids such as blood and a dialysate. That is, the vessel occupies most of the bioartificial renal tubule according to the present invention excluding the artificial hollow fiber membrane.

Brief Description of Drawings

[Fig. 1]
Fig. 1 is a schematic view of a bioartificial kidney. In this figure, A and V represent an artery and a vein, respectively.
[Fig. 2]

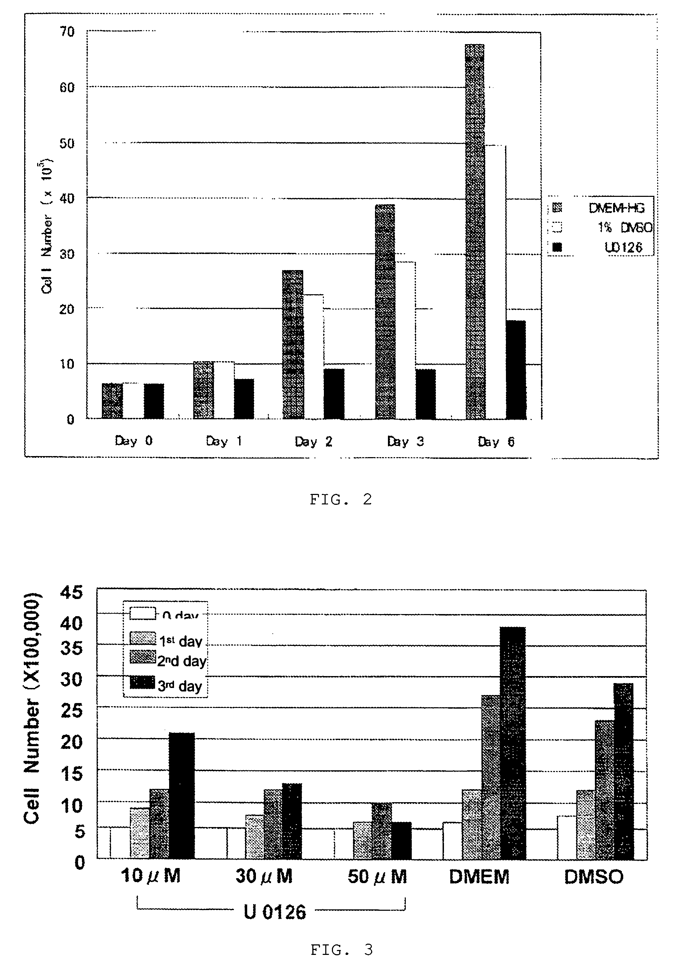

Fig. 2 shows the change in number of LLC-PK$_1$ cells cultured in (1) a DMEM-high glucose (DMEM-HG) medium, (2) a DMEM-HG medium containing 1% DMSO, or (3) a DMEM-HG medium containing 50 $\mu$M U0126 and 1% DMSO from 0 day (Day 0) to the sixth day (Day 6).
[Fig. 3]
Fig. 3 shows the change in number of LLC-PK$_1$ cells cultured in (1) a DMEM-HG medium containing 10 $\mu$M U0126 and 1% DMSO, (2) a DMEM-HG medium containing 30 $\mu$M U0126 and 1% DMSO, (3) a DMEM-HG medium containing 50 $\mu$M U0126 and 1% DMSO, (4) a DMEM-HG medium, or (5) a DMEM-HG medium containing 1% DMSO from 0 day to the third day.
[Fig. 4]

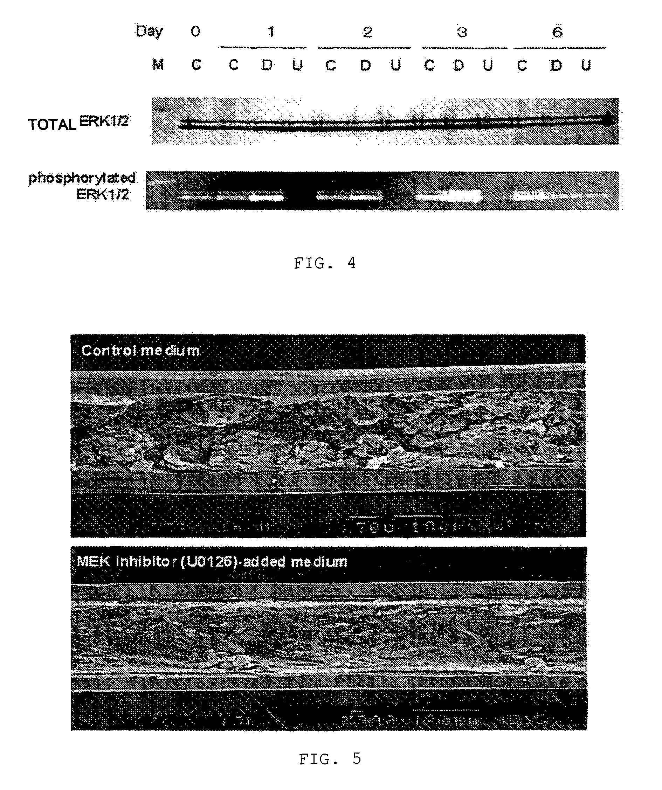

Fig. 4 shows bands obtained by subjecting total ERK1/2 (upper row) and activated ERK1/2 (lower row) to Western blot analysis after LLC-PK$_1$ cells formed confluent monolayers (0 day). Shown is the amount of proteins in ERK1/2 of the LLC-PK$_1$ cells cultured in a DMEM-HG medium (C), a DMEM-HG medium containing 1% DMSO (D), and a DMEM-HG medium containing 50 $\mu$M U0126 and 1% DMSO (U) on the first day, the second day, the third day, or the sixth day from the left.
[Fig. 5]
The following groups were checked by scanning electron microscopy: groups (three examples) in which LLC-PK$_1$ cells that were seeded in lumens of ethylene-vinyl alcohol (EVAL) copolymer membrane mini-modules at a density of $2.2 \times 10^8$ cells/ml and were then cultured in a DMEM medium for two weeks and groups (three examples) in which those cultured in a DMEM medium containing 50 $\mu$g/ml U0126 for two weeks. In the groups of the cells cultured in the DMEM medium, the lumens are narrowed because of the stratification of the cells (upper region). In the groups of the cells cultured in the U0126-containing DMEM medium, confluent monolayers are substantially maintained (lower region).
[Fig. 6] Fig. 6 shows a cross-sectional SEM image of a hollow fiber placed in an EVAL hollow fiber module in which primary cultured human proximal renal tubular epithelial cells RPTEC were seeded at a density of $1 \times 10^7$ /ml and were then cultured in a DMEM-HG medium for six days (upper image) and for 14 days (lower image). On the sixth day, the cells are stacked on a monolayer (a portion indicated by an arrow in the upper image). On the fourteenth day, clusters of the cells narrow a lumen of a hollow fiber and are dead and the lumen is partly blocked.

Best Modes for Carrying Out the Invention

Current dialysis treatments using artificial kidneys intermittently and incompletely carry out the filtration function of glomeruli. The artificial kidneys are used for dialysis for 12 hours (about 7%) in 168 hours per week only and are far inferior to vital kidneys in ability to excrete water and metabolites. Therefore, maintenance hemodialysis patients are severely restricted in water and food intake and suffer from various complications due to such incompleteness. The complications increase treatment costs such as hospital charges and surgical costs year after year to contribute to the increase in national medical costs.

In order to improve the efficiency of hemodialysis treatments, the severe restriction factor of 12 hours per week unavoidably needs to be eliminated. On the other hand, the patients spent important daytime hours for hospital visits and treatments every other day, and current dialysis techniques are practically incapable of increasing treatment hours. In order to resolve the contradiction between the necessity for extended treatment hours for achieving a treatment efficiency close to that of kidneys and the protection of the freedom of the patients, technical breakthroughs need to be made in treatment systems.

A bioartificial renal tubule according to the present invention, as well as a bioartificial glomerulus, made by the inventors (Saito A, Research in the development of a wearable bioartificial kidney with a continuous hemofilter and a bioartificial tubule device using tubular epithelial cells, Artif. Organs, 2004, 28, 58-6), will probably play a pioneering role in developing a complete artificial kidney (Fig. 1). That is, the bioartificial renal tubule according to the present invention is used in combination with the bioartificial glomerulus with forming an antithrombogenic circuit. This enables continuous hemodialysis and the selective reabsorption of useful substances in the field of next-generation artificial kidney treatments such as ambulatory artificial kidney treatments and dialysate regeneration-type peritoneal dialyses.

For the development of bioartificial glomeruli having not only high blood compatibility and high antithrombogenicity but high filtration performance, large fenestrae need to be formed in such a manner that confluent cell monolayers are formed on the inner surfaces of filtration membranes used in hemofilters using vascular endothelial cells of patients. The bioartificial glomerulus made by the inventors enables the increase of the number and size of pores (windows), called fenestrae, present in the cell membranes of glomerular endothelial cells and also enables the prolongation of duration depending on the use of an actin filament inhibitor such as Cytochalasin B. This is important in maintaining the dialysis efficiency of bioartificial kidneys.

Two types of liquids discharged through separate outlets arranged in the bioartificial glomerulus are fed through the next device, that is, the bioartificial renal tubule as shown in Fig. 1. Filtered blood is introduced into the bioartificial renal tubule according to the present invention through a side surface thereof. The blood flows outside a hollow fiber placed in the bioartificial renal tubule and gets substances reabsorbed through lumens of the hollow fiber. The blood drains from another portion of the side surface and returns to patient's venous blood stream. On the other hand, a plasma filtrate (dialysate) discharged from a side surface of the bioartificial glomerulus is an ultrafiltrate and contains various low-molecular-weight substances and electrolytes passing through a hollow fiber filtration membrane. The filtrate is fed through an inner portion (lumen) of the hollow fiber of the bioartificial renal tubule. A serum filtrate drains from another bottom surface thereof and is eliminated in the form of "urine". Useful substances, water, electrolytes, and the like are reabsorbed when passing inside the bioartificial renal tubule and are then taken into blood flowing outside the hollow fiber.

An exemplary system is a continuous wearable bioartificial kidney in which the bioartificial glomerulus and the bioartificial renal tubule are interlocked with a rotary pump through an antithrombogenic circuit and which is continuously operable for a long period. The inventors have prepared this type of artificial kidney, and have proved that the artificial kidney is capable of maintaining the contents of low-molecular-weight substances such as urea, creatinine, and uric acid as well as the content of $\beta_2$-microglobulin, which is a precursor protein causing dialysis amyloidosis, at extremely low values as compared to current hemodialyses when continuously filtering 10 litters (L) of blood per day with the artificial kidney (Saito A, Takagi T, Sugiura S, Ono M, Minakuchi K, Teraoka S, and Ota K, Maintaining low concentration of plasma $\beta$2-microglobulin through continuous slow haemodialysis, Nephrol. Dial. Transplant. 10 (Suppl. 3), 52-56, 1995). The continuous filtration of 10 L of blood per day corresponds to the continuous removal of a filtrate from blood at a rate of about 7 ml/min. Hence, the artificial kidney need not be a common-use hollow fiber module having a membrane area of about 1.8 $m^2$ but may be a filtration module which has a membrane area of 0.2 to 0.3 $m^2$ and which can be contained in a breast pocket. This minimizes the burden of carrying the artificial kidney (Saito A, Research in the development of a wearable bioartificial kidney with a continuous hemofilter and a bioartificial tubule device using tubular epithelial cells, Artif. Organs, 2004, 28, 58-6). The artificial kidney, as well as a vital kidney, is capable of immediately filtering off ingested water and produced metabolites, which are therefore not stored in a body overnight. Hence, the artificial kidney is low in physical stress and hardly causes complications. In order to allow such continuous ambulatory hemodialysis, a single hollow fiber filtration device (bioartificial glomerulus) needs to work for one week or more and the bioartificial renal tubule needs to work for about one month even though systemic anticoagulant therapy is minimized. A single current continuous hemofilter using an artificial material needs to work only for up to 24 hours after systemic anticoagulant therapy is performed.

An ideal system is supposed to be an implantable bioartificial kidney that continuously works with self-blood pressure for a long period in a pumpless mode.

Examples

Usable materials specified herein are merely preferred examples that are within the scope of the present invention. The names of devices used in examples below and numerical conditions such as the concentration, amount, treatment time, and treatment temperature of a material used in each example are also merely preferred examples that are within the scope of the present invention. Several drawings are used for descriptions below. These drawings are merely illustrated schematically such that the present invention can be understood.

In this study, five searches below were performed to investigate the effect of the MEK inhibitor U0126 on the formation and maintenance of confluent monolayers of proximal renal tubular epithelial cells.

[Example 1]

- Method

On each transwell filter unit, $2 \times 10^6$ Lewis-lung cancer porcine kidney (LLC-PK$_1$) cells, which were cryopreserved porcine proximal renal tubular epithelial cell lines, were seeded. The LLC-PK$_1$ cells were cultured in (1) a high-glucose -DMEM (DMEM-HG) medium, (2) a DMEM-HG medium containing 1% dimethyl sulfoxide (DMSO), or (3) a DMEM-HG medium, containing 50 $\mu$M U0126 and 1% DMSO, placed in outside of inserts (lumens were filled with the DMEM-HG medium). The LLC-PK$_1$ cells on 0 day, the first day, the second day, the third day, and the sixth day after seeding were colored with trypan blue and then measured for number for each group. The LLC-PK$_1$ cells were gently washed with a sterilized PBS solution containing no magnesium or calcium and were then trypsin-treated with 1 ml of a trypsin-EDTA solution at 37°C for 15 minutes. The treated cells (1 ml) were added to 4 ml of DMEM, were colored with trypan blue, and were then measured for number with a hemocytometer. Measurement was performed three times for each group.

- Results

Fig. 2 shows the change in number of the LLC-PK$_1$ cells cultured in (1) the DMEM-HG medium, (2) the DMEM-HG medium containing 1% DMSO, or (3) the DMEM-HG medium containing 50 $\mu$M U0126 and 1% DMSO from 0 day to the sixth day. As is clear from this figure, the cells cultured in these media increase in number with day; however, the cells cultured in the U0126-containing medium are prevented from significantly increasing in number.

[Example 2]

- Method

On each 6-well plate, $5 \times 10^5$ LLC-PK$_1$ cells were seeded. Broths used to culture the cells were replaced with the following three media 24 hours later after seeding: (1) a high-glucose DMEM (DMEM-HG) medium containing 10 $\mu$M U0126, which is a MEK inhibitor, and 1% DMSO; (2) a DMEM-HG medium containing 30 $\mu$M U0126 and 1% DMSO 1%; and (3) a DMEM-HG medium containing 50 $\mu$M U0126 and 1% DMSO. Furthermore, two groups of the cells cultured in (4) a DMEM-HG medium and (5) a DMEM-HG medium containing 1% DMSO were checked. The cells on 0 day, the first day, the second day, and the third day after seeding were colored with trypan blue and then measured for number.

- Results

Fig. 3 shows the change in number of the LLC-PK$_1$ cells grown in the media (1) to (5) from 0 day to the third day. In the 1% DMSO-containing medium group, the increase of the cells is suppressed to some extent as compared to in the DMEM-HG medium group, but the cells clearly increase in number with day. The cells of the 10 $\mu$M U126-containing medium group are not sufficiently prevented from increasing in number with day; however, the cells of the 30 $\mu$M and 50 $\mu$M U126-containing medium groups are clearly prevented from proliferating.

[Example 3]

- Method

In each of 6-well plates, $5 \times 10^5$ LLC-PK$_1$ cells were seeded. The cells were divided into three groups on the following day (0 day). That is, the cells of each group were cultured in a corresponding one of the following three media for three days: (1) a DMEM-HG medium (C) used as a control, (2) a DMEM-HG medium containing 1% DMSO (D), and (3) a DMEM-HG medium containing 50 $\mu$M U0126 and 1% DMSO (U). Then the media (D) and (U) were replaced with a DMEM-HG medium. The cells of each group were further cultured for three days. The cells were subjected to Western blot analysis for ERK1/2 on 0 day, the first day, the second day, the third day, and the sixth day.

The cultured LLC-PK$_1$ cells were used in the form of a cytolytic solution for Western blot analysis. The sampled cells of each group were rinsed with ice-chilled PBS and were then centrifuged at 4°C for five minutes at 1500 rpm. The centrifugate was collected and then maintained at -80°C.

Proteins were taken from 50 $\mu$L of each of ice-chilled cytolytic solutions containing 20mM Tris-HCl (pH 7.5), 150 mM NaCl, 1 mM Na$_2$EDTA, 1 mM EGTA, 1% Triton, 2.5 mM sodium pyrophosphate, 1 mM $\beta$-glycerophosphate, 1mM Na$_3$VO$_4$ (sodium orthovanadate), and 1 $\mu$g/mL leupeptin. The proteins were centrifuged at 4°C for 15 minutes at 15000 $\times$ g, whereby soluble proteins were extracted. The protein concentration of each cytolytic solution was measured with a protein assay kit (Protein Assay Rapid Kit; Wako, Osaka, Japan). A Laemmli buffer containing mercaptoethanol was added to the cytolytic solutions such that the diluted cytolytic solutions had the same concentration. The cytolytic solutions were heated at 100°C for five minutes. Proteins (20 $\mu$g) in each LLC-PK$_1$ cytolytic solution were analyzed with SDS-PAGE (polyacrylamide gel electrophoresis) using a 12.5% polyacrylamide gel (e-PAGEL; ATTO, Tokyo, Japan). The proteins were transferred on polyvinylidene difluoride (PVDF) films (BIO-RAD, Hercules, CA) and were then treated with PBS containing fat-free skim milk, 0.1% sodium azide, and 0.1% Tween-20 for 60 minutes for the purpose of reducing the amount of nonspecific antibodies. After the films were treated with a p44/42 (ERK1/2) MAPK antibody or a phospho-p44/42 MAPK antibody (1:1,000; Cell Signaling Technology), total ERK1/2 and activated ERK1/2 were detected with an enhanced chemiluminescence HRP Western blot detection system (LumiGLO reagent, Cell Signaling Technology) using a probe of 2,000-times-diluted horseradish peroxidase (HRP)-linked anti-rabbit IgG.

- Results

Fig. 4 shows culture conditions of ERK1/2 subjected to Western blot analysis after the LLC-PK$_1$ cells formed confluent monolayers (0 day) and also shows daily changes during culture. The upper row of this figure shows the expression of total ERK1/2 and the lower row of this figure shows the expression of activated ERK1/2. Three Western blots are arranged at each of positions corresponding to the first day, the second day, the third day, and the sixth day in a left-to-right manner. The blots of each of the first day, the second day, the third day, and the sixth day show the expression of an ERK1/2 protein obtained by culture with the DMEM-HG medium (C), that with the DMEM-HG medium containing 1% DMSO (D), and that with the DMEM-HG medium containing 50 $\mu$L U0126 and 1% DMSO (U) arranged from the left. Samples taken on 0 day and the sixth day and control (C) samples taken on the first day, the second day, and the third day are results obtained by culture with the DMEM-HG medium. Although the amount of ERK1/2 obtained by U0126 treatment (U) is hardly different from that obtained by DMSO treatment (D) as shown in the upper row, activated ERK1/2 in each of U0126-treated samples (U) taken on the first day, the second day, and the third day is suppressed (the lower row). In DMSO-treated samples (D), the expression thereof is suppressed or promoted in contrast. Activated ERK1/2 is expressed in a U0126-treated sample (U) taken on the sixth day; hence, the action of U0126 is supposed to be reversible.

[Example 4]

- Method

In order to confirm whether a confluent monolayer of LLC-PK$_1$ cells can be maintained on the inner surface of a hollow fiber module using U0126, $2.2 \times 10^8$ LLC-PK$_1$ cells were seeded in modules (manufactured by Kuraray, Tokyo) which had a membrane area of 65 cm$^2$ and which included hollow fibers made of an ethylene-vinyl alcohol (EVAL) copolymer and were then cultured using a DMEM-HG medium, whereby confluent monolayers were formed. On the next day, the modules were divided into two groups: one in which the DMEM-HG medium was replaced with a medium containing 50 $\mu$M U0126 and 1% DMSO and the cells were cultured using this medium, and another in which the cells were cultured using the former medium, which contained no U0126 or DMSO. In both groups, the medium was circulated outside the hollow fibers of the module in a closed system. On the third day, the media of both groups were replaced with a medium containing no U0126 or DMSO. On the fourth day after culture, three of the modules of each group were fixed with glutaraldehyde and were then observed in vertical cross section by scanning electron microscopy (SEM) .

- Results

Typical examples of both groups are shown in Fig. 5. In the U0126-containing culture solution group, confluent monolayers are substantially maintained as shown in a lower region of this figure. In the U0126-free group, lumens of the hollow fibers are narrowed because of the progress of stratification and swelling as shown in an upper region of this figure.

[Example 5]

- Method

In lumens of hollow fibers made of an ethylene-vinyl alcohol (EVAL) copolymer, which forms membranes placed in mini-modules (a membrane area of 65 cm$^2$) , $2 \times 10^8$ Lewis-lung cancer porcine kidney (LLC-PK$_1$) cells, which were porcine proximal renal tubular epithelial cell lines, were seeded. The cells were cultured in such a manner that a DMEM-HG medium was circulated inside and outside the hollow fibers at a rate of 0.25 ml/min. In this operation, the transmembrane pressure was maintained at zero. On the first day after seeding, the DMEM-HG medium circulated outside the hollow fibers in an experiment group (four) was replaced with a DMEM-HG medium containing 50 $\mu$M U0126. The former DMEM-HG medium containing no U0126 or DMSO was circulated in a control group (four). On the third day after seeding, a DMEM-HG broth containing 50 mg/dl urea nitrogen (UN) and 5 mg/dl creatinine (Cr) was fed inside the hollow fibers at a rate of 0.25 ml/min and a DMEM-HG broth containing no urea or creatinine was fed outside the hollow fibers at that rate. The leakage of urea nitrogen, the leakage of creatinine, the amount of reabsorbed glucose, and the amount of reabsorbed Na$^+$ were measured for 24 hours.

Method of measuring absorption and leakage: the absorption and leakage are calculated using the following formula:

$$\text{Absorption rate (leaking rate)}$$

$$= (C_0 \times V_0 - C_I \times (V_I - V_R)) / C_2 V_2$$

wherein $C_0$ represents the concentration of a substance in a discharged external solution, $V_0$ represents the volume of the discharged external solution, $C_I$ represents the concentration of the substance in a fed external solution, $V_I$ represents the volume of the fed external solution, $V_R$ represents the volume of the external solution remaining outside the hollow fibers at the end of an experiment, $C_2$ represents the concentration of the substance in a fed internal solution, and $V_2$ represents the volume of the fed internal solution.

- Results

Results shown in Table 1 were obtained. The leakage of urea nitrogen (UN) in a U0126 group is significantly less than that in a control group and the amount of reabsorbed Na$^+$ in the U0126 group is significantly greater than that in the control group. The leakage of creatinine (Cr) in the U0126 group is less than that in the control group, though there is no significant difference therebetween. The amount of reabsorbed glucose in the U0126 group is greater than that in the control group, though there is no significant difference therebetween.

These results suggest that the use of U0126 maintains the contact inhibition of the proximal renal tubular epithelial cells, increases the adhesion between cells to reduce leakage, and prevents cell layers from being formed on cells to maintain the active transport of glucose and Na$^+$.

Fig. 6 shows SEM images of layers of primary cultured human proximal renal tubular epithelial cells RPTEC placed in an EVAL hollow fiber. The primary cultured human proximal renal tubular epithelial cells RPTEC were seeded in an EVAL hollow fiber module at a density of $1 \times 10^7$/ml and were then cultured in a medium. One of the cross-sectional SEM images of hollow fibers was taken after the cells were cultured for six days (upper image) and the other one was taken after the cells were cultured for 14 days (lower image). The cells cultured for six days are stacked on a monolayer (a portion indicated by an arrow in the upper image). Clusters of the cells cultured for 14 days narrow a lumen of the hollow fiber and the lumen is partly blocked.

Table 1

| | Leakage (%) | | Reabsorption | |
|---|---|---|---|---|
| | UN | Cr | Glucose (mg/day) | Na$^+$ (mEq/day) |
| Control group (without U0126) | 11.9$\pm$4.2 | 9.2$\pm$3.9 | 51.3$\pm$12.0 | 0.36$\pm$0.20 |
| U0126-treated group | 3.6$\pm$0.7[b] | 3.3$\pm$0.5 | 63.9$\pm$19.7 | 1.26$\pm$0.10 [b] |
| Experiments were performed on days 3-4. Data are presented as means $\pm$ standard errors of mean: n = 4 each<br>[b] Significant difference (p<0.05) from the control group | | | | |

Industrial Applicability

The bioartificial renal tubule according to the present invention is a device which forms an artificial kidney together with a bioartificial glomerulus. The bioartificial renal tubule is useful in providing a safe, simple sustainable treatment system that prevents a disadvantage due to the continuous use of an anticoagulant agent such as heparin when blood needs to be continuously filtered to improve symptoms, such as chronic or acute cardiac failure, chronic or acute renal failure, and multiple organ failure, causing overhydration or accumulation of metabolites.

**Claims**

1. A bioartificial renal tubule comprising an artificial membrane having renal tubular epithelial cells attached to the inner or outer surface thereof and a vessel containing the artificial membrane, **characterized in that** the cells are prevented by the use of a MEK inhibitor from being stratified and therefore form a confluent monolayer on the artificial membrane.

2. The bioartificial renal tubule according to Claim 1, wherein the renal tubular epithelial cells exhibit contact inhibition because of the use of the MEK inhibitor.

3. The bioartificial renal tubule according to Claim 1 or 2, wherein the renal tubular epithelial cells are RPTEC or other primary cultured renal tubular epithelial cells, MDCK cells, LLC-PK$_1$ cells, JTC-12 cells, or HK-2 cells.

4. The bioartificial renal tubule according to any one of Claims 1 to 3, wherein the MEK inhibitor is at least one selected from the group consisting of U0126, PD98059, and CI-1040.

5. The bioartificial renal tubule according to Claim 4, wherein the MEK inhibitor is U0126.

6. The bioartificial renal tubule according to any one of Claims 1 to 5, wherein the artificial membrane is formed from a hollow fiber membrane formed from a film made of polysulfone, cellulose acetate, polyimide or an ethylene vinyl-lalcohol copolymer.

7. A method of maintaining a confluent monolayer of renal tubular epithelial cells in a bioartificial renal tubule, comprising applying a MEK inhibitor to renal tubular epithelial cells such that the cells exhibit contact inhibition and preventing the stratification of the cells after the cells form the confluent monolayer on the inner an outer surface of the artificial membrane.

8. The method according to Claim 7, wherein the MEK inhibitor is used in such a manner that the MEK inhibitor is added to a maintenance broth for culturing the renal tubular epithelial cells such that the concentration of the MEK inhibitor in the maintenance broth is 30 to 50 $\mu$M.

**Patentansprüche**

1. Bioartifizieller Nierentubulus umfassend eine künstliche Membran, die epitheliale Zellen des Nierentubulus an ihrer inneren oder äußeren Oberfläche angelagert hat, und ein die künstliche Membran enthaltendes Gefäß, **dadurch gekennzeichnet, dass** die Zellen durch die Verwendung eines MEK-Inhibitors davor bewahrt werden, aufgeschichtet zu werden und daher eine konfluente Monoschicht auf der künstlichen Membran bilden.

2. Der bioartifizielle Nierentubulus gemäß Anspruch 1, wobei die epithelialen Zellen des Nierentubulus Kontakthemmung durch die verwendung des MEK-Inhibitors besitzen.

3. Der bioartifizielle Nierentubulus gemäß Anspruch 1 oder 2, wobei die epithelialen Zellen des Nierentubulus RPTEC oder andere primär kultivierte epitheliale Zellen des Nierentubulus, MDCK Zellen, LLC-PK$_1$ Zellen, JTC-12 Zellen oder HK-2 Zellen sind.

4. Der bioartifizielle Nierentubulus gemäß einem der Ansprüche 1 bis 3, wobei der MEK-Inhibitor zumindest einer ist, der aus der Gruppe ausgewählt ist, die aus U0126, PD98059 und CI-1040 besteht.

5. Der bioartifizielle Nierentubulus gemäß Anspruch 4, wobei der MEK-Inhibitor U0126 ist.

6. Der bioartifizielle Nierentubulus gemäß einem der Ansprüche 1 bis 5, wobei die künstliche Membran aus einer Hohlfasermembran gebildet ist, die aus einer aus Polysulfon, Celluloseacetat, Polyimid oder einem Ethylen-Vinylalkohol-Copolmer hergestellten Folie hergestellt wurde.

7. Verfahren zur Erhaltung einer konfluenten Monoschicht epithelialer Zellen des Nierentubulus in einem bioartifiziellen Nierentubulus, umfassend das Applizieren eines MEK-Inhibitors auf epitheliale Zellen des Nierentubulus, so dass die Zellen Kontakthemmung besitzen, und Verhindern der Aufschichtung der Zellen nachdem die Zellen eine konfluente Monoschicht auf der inneren oder äußeren Oberfläche der künstlichen Membran bilden.

8. Das Verfahren gemäß Anspruch 7, wobei der MEK-Inhibitor derart verwendet wird, dass der MEK-Inhibitor zu einem Erhaltungsmedium für die Kultivierung der epithelialen Zellen des Nierentubulus zugegeben wird, so dass die Konzentration des MEK-Inhibitors in dem Erhaltungsmedium 30 bis 50 $\mu$M beträgt.

**Revendications**

1. Tubule rénal bioartificiel comprenant une membrane artificielle ayant des cellules épithéliales tubulaires rénales attachées à la surface intérieure ou extérieure de cette dernière et un conteneur contenant la membrane artificielle, **caractérisé en ce que** les cellules sont empêchées d'être stratifiées par l'utilisation d'un inhibiteur de MEK et forment par conséquent une monocouche confluente sur la membrane artificielle.

2. Tubule rénal bioartificiel selon la revendication 1, dans lequel les cellules épithéliales tubulaires rénales présentent une inhibition de contact à cause de l'utilisation de l'inhibiteur de MEK.

3. Tubule rénal bioartificiel selon la revendication 1 ou 2, dans lequel les cellules épithéliales tubulaires rénales sont des cellules RPTEC ou d'autres cellules épithéliales tubulaires rénales cultivées primaires, des cellules MDCK, des cellules LLC-PK$_1$, des cellules JTC-12, ou des cellules HK-2.

4. Tubule rénal bioartificiel selon l'une quelconque des revendications 1 à 3, dans lequel l'inhibiteur de MEK est au moins un inhibiteur sélectionné à partir du groupe constitué par U0126, PD98059, et CI-1040.

5. Tubule rénal bioartificiel selon la revendication 4, dans lequel l'inhibiteur de MEK est U0126.

6. Tubule rénal bioartificiel selon l'une quelconque des revendications 1 à 5, dans lequel la membrane artificielle est formée d'une membrane de fibre creuse formée d'un film fait de polysulfone, d'acétate de cellulose, de polyimide ou d'un copolymère d'éthylène - alcool vinylique.

7. Procédé d'entretien d'une monocouche confluente de cellules épithéliales tubulaires rénales dans un tubule rénal bioartificiel, comprenant l'application d'un inhibiteur de MEK aux cellules épithéliales tubulaires rénales de sorte que les cellules présentent une inhibition de contact, et la prévention de la stratification des cellules après que les cellules forment la monocouche confluente sur la surface intérieure ou extérieure de la membrane artificielle.

8. Procédé selon la revendication 7, dans lequel l'inhibiteur de MEK est utilisé de telle sorte que l'inhibiteur de MEK est ajouté à un milieu liquide d'entretien pour la culture des cellules épithéliales tubulaires rénales de sorte que la concentration de l'inhibiteur de MEK dans le milieu liquide d'entretien est de 30 à 50 $\mu$M.

FIG. 1

FIG. 2

FIG. 3

FIG. 4

FIG. 5

FIG. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2004267795 A
- WO 9511048 A

- JP 2916446 B

### Non-patent literature cited in the description

- **Aebischer P ; Ip TK ; Miracoli L ; Galletti PM.** Renal epithelial cells grown on semipermeable processor. *Trans. Am. Soc. Artif. Intern. Organs,* 1987, vol. 33, 96-102
- **Aebischer P ; Ip TK ; Miracoli L ; Galletti PM.** The bioartificial kidney: Progress toward an ultrafiltration device with renal epithelial cells processing. *Life Support Sys.,* 1987, vol. 5, 159-168
- **Ip TK ; Aebischer P.** Renal epithelial cell-controlled solute transport across permeable membranes as the foundation for a bioartificial kidney. *Artif. Organs,* 1987, vol. 13, 58-61
- **Humes HD ; MacKay SM ; Funke AJ ; Buffington DA.** Tissue engineering of a bioartificial renal tubule assist device: in vitro transport and metabolic characteristics. *Kidney Int.,* 1999, vol. 55, 2502-2514
- **Humes HD ; Buffington DA ; MacKay SM ; Funke AJ ; Weitzel WF.** Replacement of renal function in uremic animals with a tissue-engineered kidney. *Nat. Biotechnol.,* 1999, vol. 17, 451-455
- **Weitzel WF ; Fissel WH ; Humes HD.** Initial clinical experience with a human proximal tubule cell renal assist device (RAD). *J. Am. Soc. Nephrol,* 2001, vol. 12, 279A
- **Humes HD ; Weitzel WF ; Barlett RH ; Swaniker FC ; Paganini EP ; Luderer JR ; Sobota J.** Initial clinical results of the bioartificial kidney containing human cells in ICU patients with acute renal failure. *Kidney Int.,* 2004, vol. 66, 1587-1588

- **Fujita Y ; Kakuta T ; Saito A et al.** Evaluation of Na+ active transport and morphological changes for bioartificial renal tubule cell device using Madin-Dar by canine kidney cells. *Tissue Eng.,* 2002, vol. 8, 13-24
- **Ozgen N ; Tarashima M ; Aung T ; Sato Y ; Isoe C ; Kakuta T ; Saito A.** Evaluation of long-term transport ability of a bioartificial renal tubule device using LLC-PK1. *Nephrol Dial Transplant,* 2004, vol. 19, 2198-2207
- **Favata MF ; Horiuchi KY ; Manos EJ ; Daulerio AJ ; Stradley DA ; Feeser WS ; Van Dyk DE ; Pitts WJ ; Earl RA ; Hobbs F.** Identification of a novel inhibitor of mitogen-activated protein kinase kinase. *J. Biol. Chem.,* 1998, vol. 273, 18623-18632
- **Li S ; Gerrard ER Jr. ; Balkovetz DF.** Evidence for ERK1/2 phosphorylation controlling contact inhibition of proliferation in Madin-Darby canine kidney epithelial cells. *Am. J. Physiol Cell Physiol,* 2004, vol. 287, C432-C439
- **Dudley DT ; Pang L ; Decker SJ ; Bridges AJ ; Saltiel AR.** *Proc. Natl. Acad. Sci. USA,* 1995, vol. 92, 7868-7889
- **Saito A.** Research in the development of a wearable bioartificial kidney with a continuous hemofilter and a bioartificial tubule device using tubular epithelial cells. *Artif. Organs,* 2004, vol. 28, 58-6
- **Saito A ; Takagi T ; Sugiura S ; Ono M ; Minakuchi K ; Teraoka S ; Ota K.** Maintaining low concentration of plasma β2-microglobulin through continuous slow haemodialysis. *Nephrol. Dial. Transplant.,* 1995, vol. 10 (3), 52-56